# EUROPEAN PATENT APPLICATION

(11) **EP 1 731 083 A1**
(43) Date of publication of application: **13.12.2006**
(21) Application number: 05728025.7
(22) Date of filing: 31.03.2005
(51) Int. Cl.: A61B 1/00

(54) **AUXILIARY TOOL FOR INSERTING MEDICAL INSTRUMENT INTO LIVING BODY**

(30) Priority: 02.04.2004 JP 2004109926; 28.03.2005 JP 2005092013
(71) Applicant: JMS Co., Ltd., Hiroshima-shi, Hiroshima 730-8652 (JP)
(72) Inventor: KOYAMA, Isamu, 3510101 (JP); HAYASHI, Shuro, JMS CO., LTD., Hiroshima-shi, Hiroshima 7308652 (JP)
(74) Representative: Laufhütte, Dieter
(86) International application number: PCT/JP2005/006348
(87) International publication number: WO 2005/094663

(57) **Abstract**

A tubular member 2 is formed of a resin material so as to have an inner passageway 2. A thin plate is shaped into a spiral along the perimeter of the tubular member 2 to form a reinforcement member 3. The reinforcement member 3 is buried in a peripheral wall 2a of the tubular member 2. The tubular member 2 and the reinforcement member 3 have curved shapes conformable to the shape of the pharynx 100. The tubular member 2 and the reinforcement member 3 are inserted from the oral cavity 102 into the pharynx 100 and retained there. A medical instrument is guided to the esophagus 103 through an inner passageway 2b of the tubular member 2.

## Description

### Technical Field

The present invention relates to a device for supporting insertion of a medical instrument into a human body, which is used for, for example, inserting a medical instrument, such as an endoscope, or the like, into a digestive organ from the oral cavity through the pharynx.

### Background Art

Conventionally, for example, in diagnosis and treatment of digestive organs, such as stomach, esophagus, etc., the manipulation of inserting a medical instrument, such as an endoscope, or the like, into a digestive organ of a patient from the oral cavity through the pharynx has been carried out. The manipulation of inserting the medical instrument into the digestive organ is difficult because the pharynx, which extends between the oral cavity and the esophagus, has a curved shape. A manipulation of repeatedly inserting and pulling out the medical instrument entails an increased number of times the medical instrument passes through the pharynx, which imposes a larger load on the patient. In view of such circumstances, for example, as disclosed in Patent Document 1, a tubular supporting device is inserted from the oral cavity through the pharynx up to the entrance of the esophagus and retained there, and the inner passageway of this supporting device is used to insert the medical instrument into and pull the medical instrument out of a digestive organ, whereby the manipulation of inserting the medical instrument is made easier, and the load on the patient is reduced.

The supporting device of Patent Document 1 is formed by a tubular member of a resin material and a reinforcement member of a spiral thin elastic wire which is buried in the tubular member. When inserted into the pharynx, this supporting member can support the inner wall of the pharynx with the wire of the reinforcement member such that the pharynx is prevented from narrowing.
Patent Document 1: Japanese Laid-Open Patent Publication No. 7-51221 (Page 3, Figure 1, Figure 4).

### Disclosure of Invention

### Problems to be solved by the invention

In recent years, the demand has been increasing that, in the diagnosis and treatment of a digestive organ, a medical instrument is inserted from the oral cavity to a digestive organ while the number and size of laparotomies and thoracotomies are decreased as small as possible or eliminated such that the load on patients is decreased. In this case, it is desirable to secure a large space for inserting the medical instrument(s) by increasing the diameter of the tubular member of Patent Document 1 because a plurality of medical instruments, including for example an endoscope for an operator to observe a subject region and an ablation tool for an affected area, are sometimes simultaneously inserted from the oral cavity, or a medical instrument having a relatively large diameter which has both the function of ablating the affected area and the endoscope function is sometimes inserted.

However, since the inner diameter of the pharynx of a patient is limited, there is a possibility that a large diameter of the tubular member makes it difficult for an operator to insert the tubular member and largely expands the pharynx of the patient, so that the load on the patient can be increased.

The present invention was conceived in view of the above circumstances. An objective of the present invention is to guide a medical instrument into a digestive organ using a tubular member inserted from the oral cavity into the pharynx and retained there while a large insertion space is secured for the medical instrument, the workability of inserting the tubular member into the pharynx is improved, and the load on the patient is reduced.

### Means for solving the problems

To achieve the above objective, according to the first invention of the present application, the reinforcement member is formed of a thin plate, and the reinforcement member and the tubular member are curved so as to be conformable to the curvature of the pharynx.

Specifically, the supporting device includes: a tubular member having an inner passageway between its opposite ends through which the medical instrument is capable of passing; and a reinforcement member formed by a thin plate extending along a perimeter of the inner passageway, wherein the tubular member and the reinforcement element have curved shapes conformable to the shape of a pharynx of a human body and, when inserted from an oral cavity into the pharynx and retained there, guide the medical instrument to a digestive organ through the inner passageway.

With this structure, the tubular member and the reinforcement member are curved according to the curvature of the pharynx. Therefore, the tubular member and the reinforcement member can be smoothly inserted into the pharynx without any substantial deformation. Thus, small force is necessary for insertion of the tubular member into the pharynx, and accordingly, the load on a patient is decreased.

Since the reinforcement member is formed by a thin plate extending along the perimeter of the inner passageway, the dimension of the reinforcement member in the thickness direction of the peripheral wall, i.e., the thickness of the thin plate, is set smaller than the wire diameter when the conventional reinforcement member is formed by a wire, so that the thickness of the peripheral wall is decreased, while the width of the thin plate is set greater than the wire diameter of the conventional wire, whereby sufficient strength of the reinforcement member can be obtained. Thus, flattening deformation of the tubular member can be suppressed while the thickness of the peripheral wall of the tubular member is decreased such that a sufficient insertion space is secured for the medical instrument.

In the second invention, a guiding member having a smaller diameter than a tubular member is inserted into the pharynx, and the tubular member can be guided to the pharynx through the guiding member.

Specifically, the supporting device includes: a tubular member having an inner passageway between its opposite ends through which the medical instrument is capable of passing; a reinforcement member extending along a perimeter of the inner passageway; and a guiding member having a diameter smaller than the inner passageway and insertable from an oral cavity of a human body into a pharynx, the guiding member guiding, when inserted into the pharynx, the tubular member and the reinforcement member from the oral cavity to the pharynx, wherein, when guided to the pharynx and retained there, the tubular member and the reinforcement member guide the medical instrument to a digestive organ through the inner passageway.

With this structure, after the guiding member is inserted from the oral cavity to the pharynx and retained there, the oral cavity end of the guiding member is inserted into the inner passageway of the tubular member, and then, the tubular member and the reinforcement member can be inserted into the pharynx along the guiding member. Specifically, the guiding member, which has a smaller diameter than the tubular member, is first inserted so that, firstly, the inside diameter of the pharynx is expanded by the guiding member, and thereafter, the inside diameter of the pharynx can be further expanded by the tubular member having a larger diameter. Such gradual expansion of the inside diameter of the pharynx can reduce the load on a human body in the process of securing a large insertion space for the medical instrument by inserting a large-diameter medical instrument into the pharynx. Further, the guiding member has a smaller diameter than the tubular member and is therefore readily inserted into the pharynx. With this guiding member, the tubular member and the reinforcement member can readily be inserted into the pharynx.

According to the third invention, in the first invention, a guiding member having a diameter smaller than the inner passageway of the tubular member and insertable from the oral cavity into the pharynx is provided. When inserted into the pharynx, the guiding member guides the tubular member and the reinforcement member from the oral cavity to the pharynx.

With this structure, the tubular member and the reinforcement member can readily be inserted into the pharynx along the guiding member as in the second invention.

According to the fourth invention, in any one of the first to third inventions, the reinforcement member has the shape of a spiral continuously extending in a center line direction of the inner passageway.

With this structure, the tubular member is continuously reinforced in the center line direction.

According to the fifth invention, in any one of the first to fourth inventions, a digestive organ end of the tubular member extends toward a digestive organ ahead of a digestive organ end of the reinforcement member.

With this structure, the end of the tubular member is closer to the digestive organ than the end of the reinforcement member is. Therefore, when inserting the tubular member and the reinforcement member into the pharynx, the reinforcement member can be prevented from coming in contact with organic tissue.

According to the sixth invention, in any one of the first to fifth inventions, the digestive organ end of the tubular member is slanted with respect to the center line of the inner passageway.

With this structure, the digestive organ end of the tubular member has a tapered shape. Therefore, the operation of inserting the tubular member into the pharynx becomes still easier.

According to the seventh invention, in any one of the first to sixth inventions, the tubular member is molded with the reinforcement member buried therein.

According to this invention, in the process of molding the tubular member, the tubular member and the reinforcement member can be integrated.

According to the eighth invention, in any one of the second to seventh inventions, the guiding member includes a guiding member engagement section; and the tubular member includes a tubular member engagement section for engaging with the guiding member engagement section of the guiding member inserted up to a predetermined position in the inner passageway of the tubular member.

According to this invention, when inserting the tubular member into the pharynx along the guiding member, the guiding member engagement section is engaged with the tubular member engagement section, so that the tubular member becomes immovable with respect to the guiding member. The guiding member has a smaller diameter than the tubular member and therefore can readily be inserted into the pharynx. Thus, the guiding member can be inserted to a correct position in the pharynx. The positions of the engagement sections are set such that, when the tubular member is inserted to a correct position in the pharynx with respect to the guiding member as a reference, the guiding member engagement section engages with the tubular member engagement section. With such setting, the operator can readily insert the tubular member to the correct position.

According to the ninth invention, in any one of the second to seventh inventions, the guiding member has a guiding member alignment mark; and the tubular member has a tubular member alignment mark aligned with the guiding member alignment mark of the guiding member inserted to a predetermined position in the inner passageway of the tubular member.

With this structure, the guiding member can be inserted to a correct position in the pharynx as in the eighth invention. The positions of the alignment marks are set such that, when the tubular member is inserted to a correct position in the pharynx with respect to the guiding member as a reference, the guiding member alignment mark engages with the tubular member alignment mark. With such setting, the operator can readily insert the tubular member to the correct position.

According to the tenth invention, in any one of the first to ninth inventions, the tubular member is made of a resin material; and the guiding member is made of another resin material harder than the resin material of the tubular member.

With this invention, flattening deformation of the guiding member inserted into the pharynx is decreased such that the pharynx can be effectively expanded. Since the tubular member, which has a larger diameter than the guiding member, is made of a resin material softer than the resin material of the guiding member, the load on a human body can be further reduced.

### Effects of the invention

According to the first invention, the reinforcement member is formed by a thin plate extending along the perimeter of the inner passageway of the tubular member. Thus, the peripheral wall of the tubular member can be made thin so that a large insertion space is secured for the medical instrument. Further, the tubular member and the reinforcement member are curved so as to be conformable to the shape of the pharynx. Therefore, the workability of insertion of the tubular member into the pharynx is improved, while the load on a human body can be suppressed.

According to the second invention, the tubular member is guided to the pharynx using the guiding member which has a smaller diameter than the tubular member. Therefore, a large insertion space can be secured for the medical instrument by inserting the tubular member having a larger diameter into the pharynx while the load on a human body is suppressed. Further, the workability of insertion of the tubular member into the pharynx is improved.

According to the third invention, the supporting device has a guiding member which has a smaller diameter than the inner passageway of the tubular member. Therefore, the workability of insertion of the tubular member and the reinforcement member into the pharynx is further improved.

According to the fourth invention, the reinforcement member has a spiral shape. Therefore, the tubular member can be continuously reinforced in the center line direction.

According to the fifth invention, the digestive organ end of the tubular member is closer to the digestive organ than the reinforcement member is. Load caused on a human body when the tubular member and the reinforcement member are inserted into the pharynx can be decreased.

According to the sixth invention, the digestive organ end of the tubular member can be tapered. Therefore, the workability of insertion of the tubular member into the pharynx is further improved.

According to the seventh invention, the tubular member and the reinforcement member can be integrated in the molding of the tubular member. Therefore, the production process can be simplified.

According to the eighth invention, when inserting the tubular member into the pharynx along the guiding member, an operator can readily insert the tubular member to a correct position.

According to the ninth invention, the tubular member can readily be inserted to a correct position as in the eighth invention.

According to the tenth invention, the pharynx can be effectively expanded by the guiding member, and the load caused on a human body when the tubular member is inserted into the pharynx can be further decreased.

### Brief Description of Drawings

FIG. **1** is a cross-sectional view of a supporting device according to embodiment 1 of the present invention.
FIG. **2** is a side view of a tubular member according to embodiment 1.
**FIG. 3** is a cross-sectional view of a tubular member according to embodiment 1.
FIG. **4** shows a tubular member retained in the larynx of a patient lying face up.
FIG. **5** is a perspective view of a reinforcement member according to embodiment 1.
FIG. **6** is a cross-sectional view of a first guiding member and second guiding member according to embodiment 1.
FIG. **7** is a side view of an anastomosis device.
**FIG. 8(a)** shows a main body of a device seen from the head side. FIG. 8(b) shows a head seen from the main body side.
FIG. **9** shows the anastomosis device with the main body inserted in the esophagus and the head inserted in the small intestine.
FIG. **10** shows an end of the esophagus anastomosed to an end of the small intestine.
FIG. **11** illustrates insertion of the main body of the anastomosis device into the esophagus using the tubular member.
FIG. **12** illustrates ablation of a stricture in the esophagus using the anastomosis device.
FIG. **13** illustrates ablation of a tumor from the stomach using an ablation instrument.
FIG. **14** shows that an endoscope, which is integrated with an ablation blade, is inserted in the stomach.
FIG. **15** illustrates ablation of early-stage stomach cancer using an ablation instrument.
FIG. **16** illustrates installation of a stent in the esophagus.
FIG. **17** is a cross-sectional view according to embodiment 2 of the present invention, which is equivalent to FIG. **1.**
FIG. **18** is a side view of a supporting device according to embodiment 2 with alignment marks being coincident with each other.
FIG. **19** is a side view of a supporting device according to a variation of embodiment 2.

### Description of Reference Numerals

- 1: Supporting Device
- 2: Tubular Member
- 2b: Inner passageway
- 2c: Cavity (Tubular Member Engagement Section)
- 3: Reinforcement Member
- 4: First Guiding Member
- 4b: Protrusion (Guiding Member Engagement Section)
- 5: Second Guiding Member
- 21: Main Body of Device (Medical Instrument)
- 100: Pharynx
- 102: Oral Cavity
- 103: Esophagus (Digestive Organ)
- 130: Tubular Member Alignment Mark
- 131, 132: First Guiding Member Alignment Mark
- 133: Second Guiding Member Alignment Mark
- 140: Cavity (Tubular Member Engagement Section)
- 141: Protrusion (Guiding Member Engagement Section)

### Best Mode for Carrying Out the Invention

Hereinafter, embodiments of the present invention will be described in detail with reference to the drawings.

### (Embodiment 1)

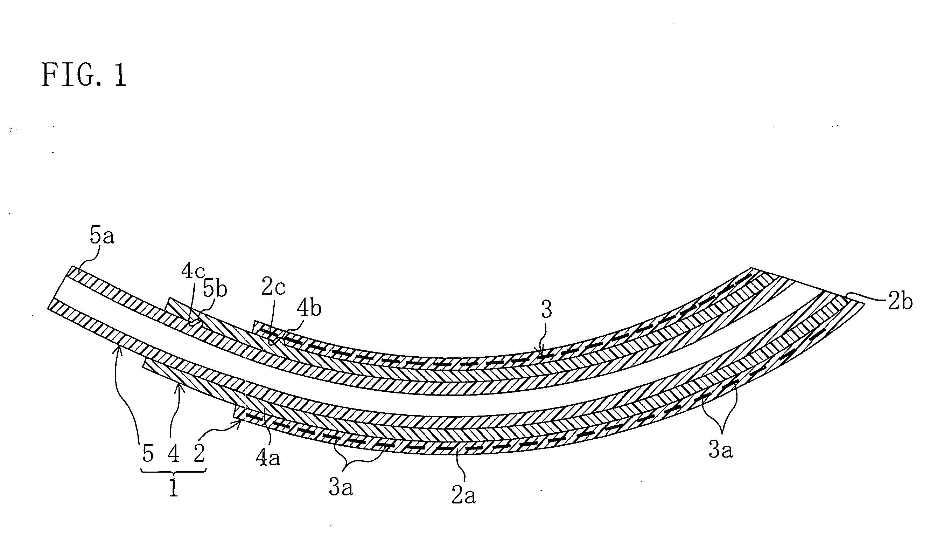

FIG. **1** is a cross-sectional view of a supporting device **1** according to embodiment 1 of the present invention for supporting insertion of a medical instrument into a human body. The supporting device **1** is to be used in inspection, diagnosis or treatment of a digestive organ, such as a human stomach, esophagus, small intestine, or the like, such that the supporting device **1** is inserted into the pharynx and retained there for insertion of the medical instrument from the oral cavity into the digestive organ.

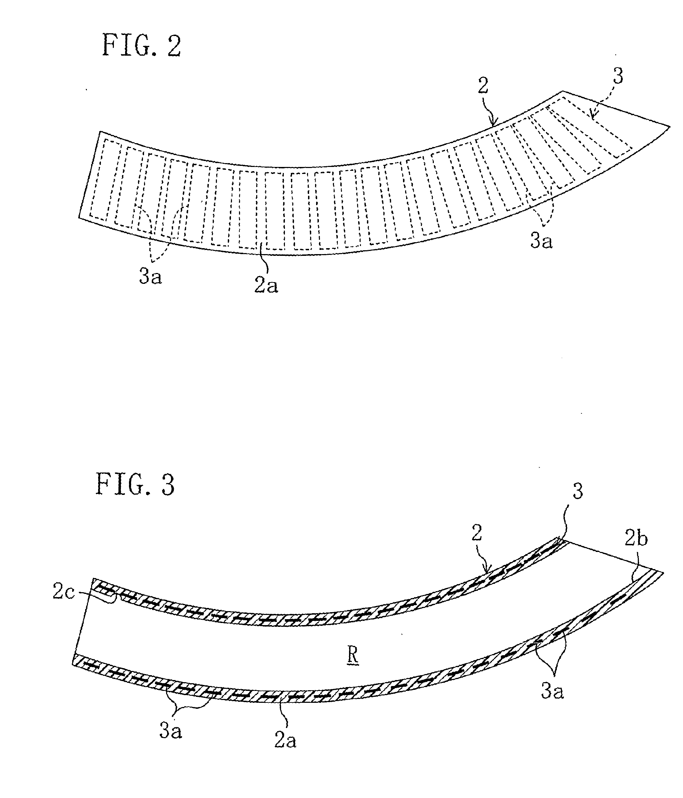

The supporting device **1** includes a tubular member **2** made of a resin, a reinforcement member **3** of a metal which is buried in a peripheral wall **2a** of the tubular member **2,** and a first guiding member **4** and second guiding member **5** which are inserted in the tubular member **2.** The tubular member **2** is formed by pouring melted polyvinyl chloride, or the like, into a mold (not shown) and solidifying the polyvinyl chloride into a cylindrical shape. As shown in FIG. **2,** FIG. 3 and FIG. **4,** the tubular member **2** has a curved shape conformable to the shape of the pharynx **100** when a human lies face up. The resin material of the tubular member **2** may be polyurethane or rubber resin.

The length of the tubular member **2** between an end closer to the oral cavity ("oral cavity end") and the other end closer to the digestive organ ("digestive organ end") is such that, for example, the oral cavity end extends from the anterior teeth to the outside of the oral cavity **102,** and the digestive organ end passes through the pharynx **100** and reaches a vicinity of the entrance of the esophagus **103.** Specifically, the length of the tubular member **2** along its center line is set between 100 mm and 300 mm.

As shown in FIG. **3,** the inner passageway **2b** of the tubular member **2** extends between the both ends of the tubular member **2** and opens at the both ends. The medical instrument is to be inserted through the inner passageway **2b.** The oral cavity end of the tubular member **2** is formed to extend in a direction generally perpendicular to the center line of the tubular member **2,** while the digestive organ end of the tubular member **2** is formed to have an inclination angle between 40° to 70° with respect to a direction in which the center line of the tubular member **2** extends. Thus, the digestive organ end of the tubular member **2** has a tapered shape.

The thickness of the peripheral wall **2a** of the tubular member **2** is set, for example, between 2 mm and 4 mm. The thickness of the peripheral wall **2a** is changed according to the thickness of a thin plate (described later) which forms the reinforcement member 3, the thickness of a resin material covering the thin plate, or the like. The inside diameter of the tubular member **2** is set between 20 mm and 30 mm. The inside diameter and longitudinal length of the tubular member **2** can be changed according to the type and size of a medical instrument which is inserted into a digestive organ using the supporting device 1, the constitution, age, or sex of a patient, etc.

The outer and inner surfaces of the tubular member **2** are formed by smooth surfaces. The outer and inner surfaces of the tubular member **2** are coated with a frictional-drag reducer. With such surfaces, the outer surface of the tubular member **2** is prevented from being caught in organic tissue when the supporting device **1** is inserted into the pharynx **100.** The medical instrument is also prevented from being caught in the inner surface of the tubular member 2 when the medical instrument is inserted into the inner passageway **2b.** The outer and inner surfaces of the tubular member **2** can be coated by applying a frictional-drag reducing agent, such as Xylocaine jelly, or the like.

As shown in FIG. **1,** the inner surface of the tubular member **2** on the oral cavity side has a cavity **2c,** while the outer surface of the first guiding member **4** on the oral cavity side has a protrusion **4b** which is fittingly engageable with the cavity 2c. The protrusion **4b** exists at such a position that, when the protrusion **4b** is engaged with the cavity **2c,** the digestive organ end of the first guiding member **4** is generally coincident with the digestive organ end of the tubular member **2.**

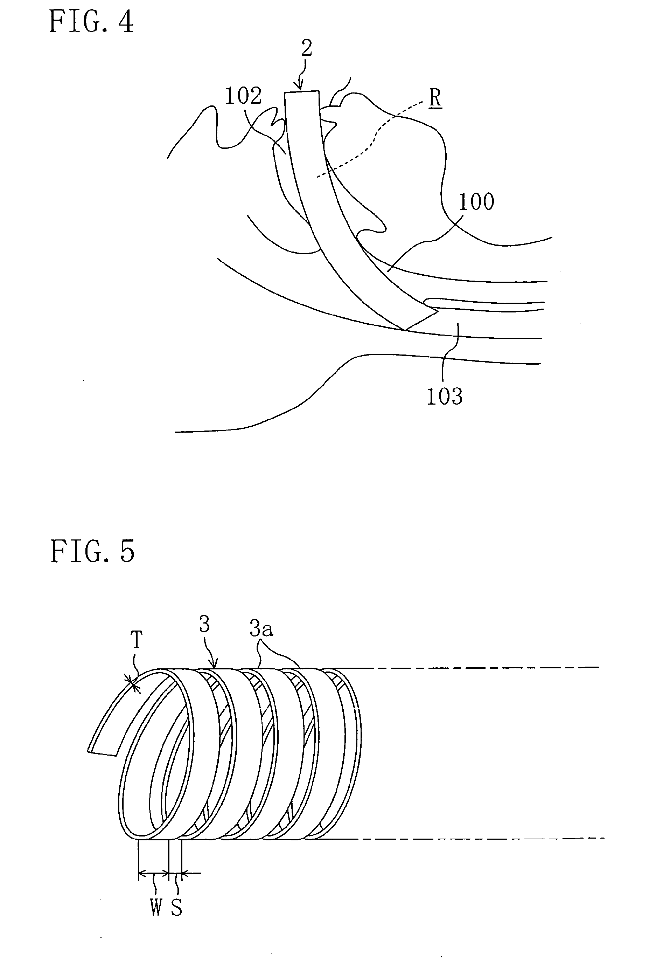

As shown in FIG. **5,** the reinforcement member **3** is formed by a long thin plate. The reinforcement member **3** is a continuous spiral along the center line of the tubular member **2,** including circular parts **3a** which extend in the peripheral direction of the inner passageway **2b** of the tubular member **2.** The length of the reinforcement member **3** along the center line is set smaller than the length of the tubular member 2 along the center line. The reinforcement member **3** is provided at an intermediate position of the tubular member **2** when located along the center line direction. Therefore, as shown in FIG. **2** and FIG. **3,** the oral cavity end of the tubular member **2** is closer to the oral cavity than the oral cavity end of the reinforcement member **3** is, and the digestive organ end of the tubular member **2** is closer to the digestive organ than the digestive organ end of the reinforcement member **3** is. Since the reinforcement member 3 has a spiral shape as described above, intermediate part of the tubular member 2 in the center line direction is continuously reinforced.

The thin plate is not limited to any particular material so long as it is elastic and has such strength that flattening deformation of the tubular member **2** can be suppressed. For example, the thin plate can be made of a metal material, such as, for example, stainless steel, etc., or a rigid resin material, such as polyamide, fluoric resin, etc. Referring to FIG. **5,** the width of the thin plate, W, is set between 1.50 mm and 5.50 mm, and the thickness of the thin plate, T, is set between 0.03 mm and 1.00 mm. In this embodiment, the thin plate is made of SUS304 where width W is about 4.00 mm and thickness T is about 0.20 mm. Making the thin plate thinner enables the thickness of the peripheral wall 2a of the tubular member 2 to be thinner but decreases the strength of the tubular member 2 against force applied in a flattening direction. In view of such, the thickness of the thin plate is preferably set between 0.05 mm and 0.50 mm. Making width W of the thin plate wider increases the strength of the tubular member **2** against force applied in a flattening direction. However, when the tubular member **2** is bent, a portion of the tubular member **2** in which the thin plate exists does not bend but keeps its linear shape. As a result, the tubular member **2** lacks smoothness in curvature, and insertion of an instrument into the pharynx **100** can be difficult. Therefore, width W of the thin plate is preferably set between 2.00 mm and 5.00 mm in consideration of the resistance to flattening deformation and the workability of insertion into the pharynx **100.**

Separation S between adjacent circular parts **3a** of the reinforcement member 3 is set to about 1.00 mm. Separation S between adjacent circular parts **3a** varies because of errors occurring in the production of the reinforcement member **3,** positional displacement of the circular parts **3a** which is caused by a melted resin material flowing in a cavity of a mold, etc. Thus, separation S is between 0.50 mm and 1.50 mm in the product specifications. Making separation S between adjacent circular parts **3a** shorter results in a denser arrangement of circular parts **3a,** which increases the strength of the tubular member **2** against force applied in a flattening direction, but results in a reduced amount of resin material dwelling between circular parts **3a,** so that it becomes difficult to deform the tubular member **2** in a curve. On the other hand, making separation S between adjacent circular parts **3a** longer results in a sparse arrangement of circular parts **3a** which decreases the strength of the tubular member **2** against force applied in a flattening direction. In view of such circumstances, separation S between circular parts **3a** is set within the above-described range.

Integration of the reinforcement member **3** having the above-described structure with the tubular member **2** is now described. First, the reinforcement member 3 is prepared in advance. Then, the reinforcement member **3** is retained in a cavity of a mold for molding the tubular member **2.** At this step, the reinforcement member 3 is deformed to have a curved shape conformable to the shape of the pharynx **100** as described above. Thereafter, melted resin material is poured in the cavity. The melted resin material flows so as to cover the outer and inner surfaces of the reinforcement member 3 and flows into a space between adjacent circular parts **3a.** The resin is then solidified. Thus, the reinforcement member **3** is insert-molded in the peripheral wall **2a.** As a result, the reinforcement member **3** is buried in the tubular member **2.**

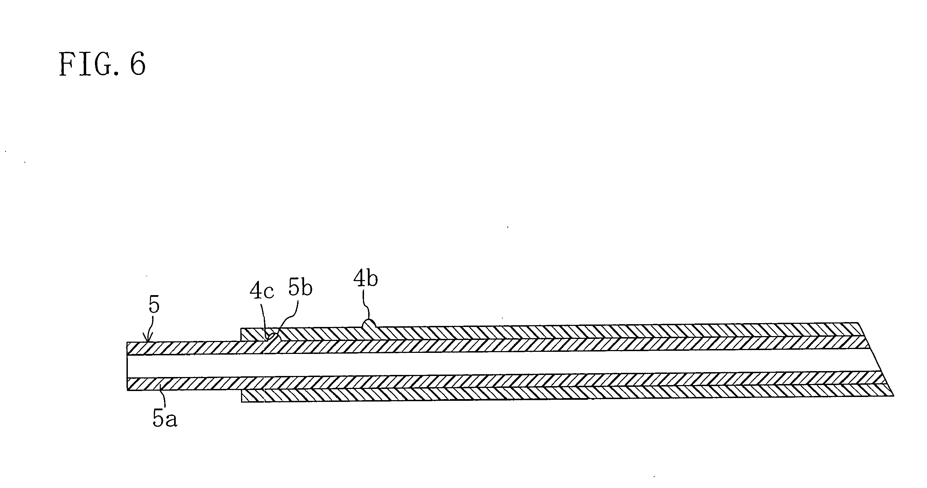

The first guiding member **4** has a tubular shape insertable into the inner passageway **2b** as shown in FIG. **1.** The second guiding member 5 has a tubular shape insertable into the first guiding member **4** as also shown in FIG. **6.** The first guiding member **4** and the second guiding member **5** are made of a resin material harder than the resin material of the tubular member 2, for example, polyethylene. As shown in FIG. **6,** the first guiding member **4** and the second guiding member 5 linearly extend. As shown in FIG. 1, the first guiding member **4** and the second guiding member **5** have such flexibility that, when inserted into the inner passageway **2b** of the tubular member **2,** the guiding members **4** and **5** are deformed in a curve conformable to the curved shape of the tubular member **2.** The outside diameter of the first guiding member **4** is set slightly smaller than the inner passageway **2b,** such that the first guiding member **4** can be readily inserted into the inner passageway **2b.** The outside diameter of the second guiding member **5** is set slightly smaller than the inside diameter of the first guiding member **4,** such that the second guiding member **5** can be readily inserted into the first guiding member **4.** The outside diameter of the second guiding member **5** is set slightly larger than the inside diameter of the pharynx **100** of a person to which the supporting device **1** is to be applied.

The thickness of the peripheral walls **4a** and **5a** of the first guiding member **4** and the second guiding member **5** is set approximately equal to that of the peripheral wall **2a** of the tubular member **2.** The length of the first guiding member 4 in the center line direction is set larger than the length of the tubular member **2** in the center line direction. The length of the second guiding member **5** in the center line direction is set larger than the length of the first guiding member **4** in the center line direction. The oral cavity ends and digestive organ ends of the first guiding member **4** and the second guiding member **5** are formed in the same way as the oral cavity end and digestive organ end of the tubular member **2.** The outer and inner surfaces of the first guiding member **4** and the second guiding member **5** may be coated as is the tubular member **2.** The first guiding member **4** and the second guiding member **5** may be formed to have a curved shape as is the tubular member **2.**

As shown in FIG. 1, the inner surface of the first guiding member 4 on the oral cavity side has a cavity **4c,** while the outer surface of the second guiding member 5 on the oral cavity side has a protrusion **5b** which is fittingly engageable with the cavity **4c.** The protrusion **5b** exists at such a position that, when the protrusion **5b** is engaged with the cavity **4c,** the digestive organ end of the first guiding member **4** is generally coincident with the digestive organ end of the second guiding member **5.**

Thus, the tubular member **2,** the first guiding member **4** and the second guiding member **5** are kept integrated by engaging the cavity **2c** of the tubular member **2** with the protrusion **4b** of the first guiding member **4** and engaging the cavity **4c** of the first guiding member **4** with the protrusion **5b** of the second guiding member **5.** This prevents, when the supporting device 1 is carried about, the first guiding member **4** and the second guiding member 5 from falling out of the tubular member **2.**

Although not shown, a protrusion may be provided on the inner surface of the tubular member **2** while a cavity engageable with the protrusion of the tubular member **2** may be provided on the outer surface of the first guiding member **4.** Further, a protrusion may be provided on the inner surface of the first guiding member **4** while a cavity engageable with the protrusion of the first guiding member **4** may be provided on the outer surface of the second guiding member **5.** The shape of the protrusions and cavities is not limited to those illustrated in the drawings.

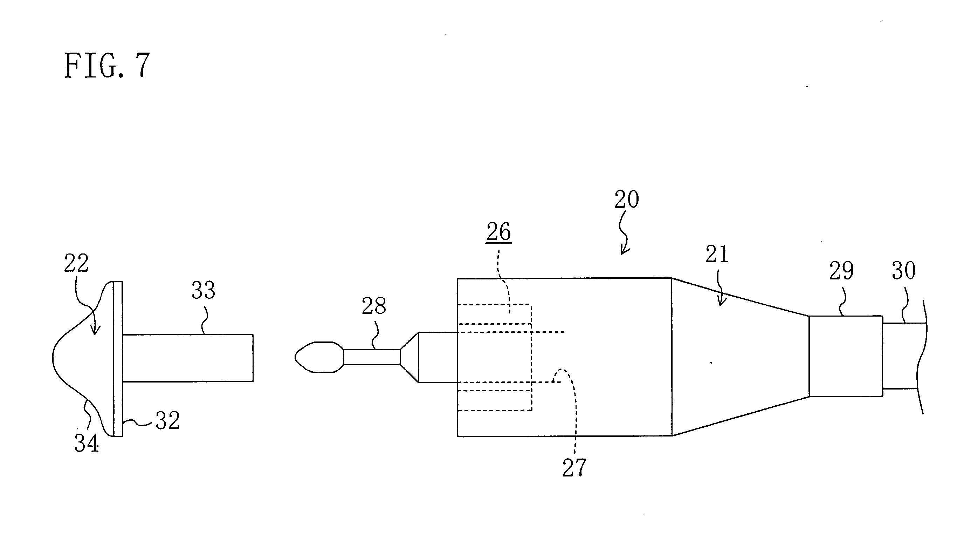

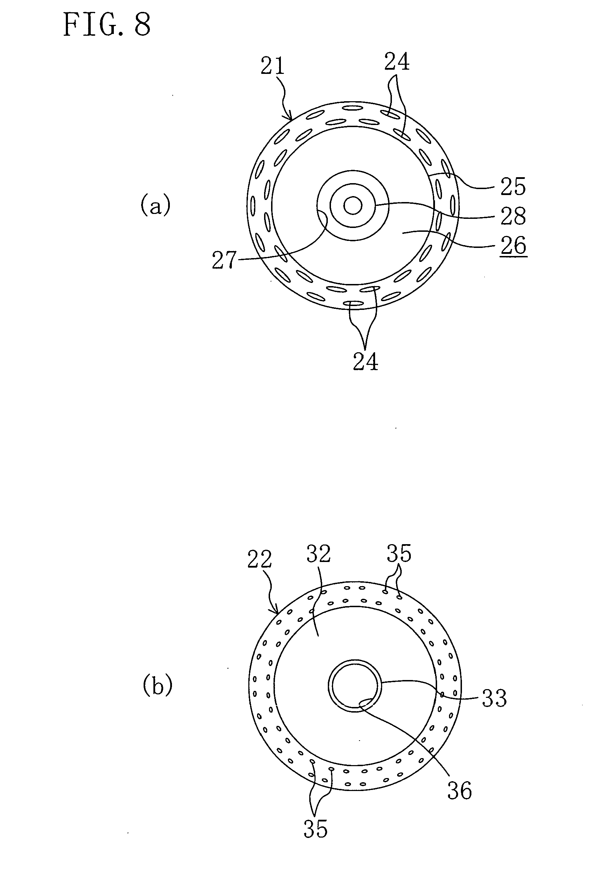

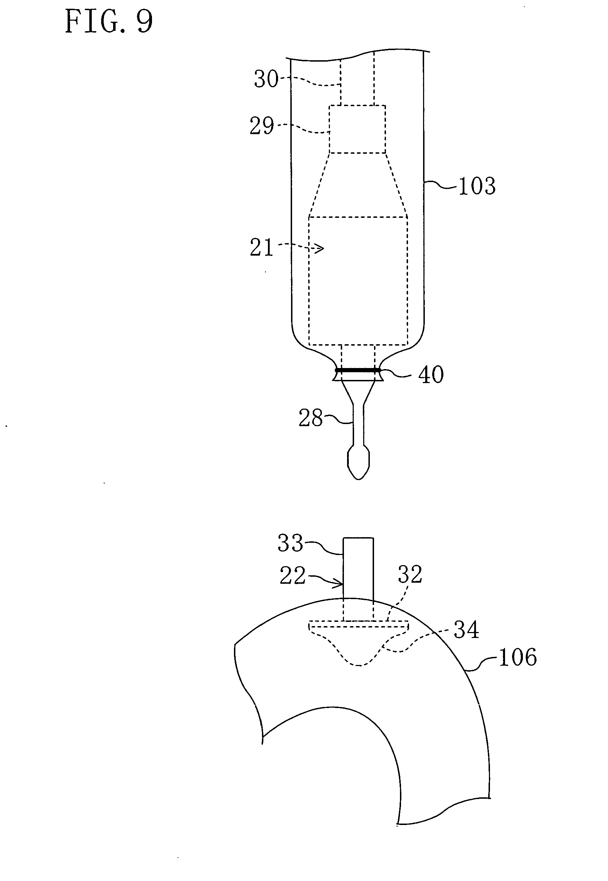

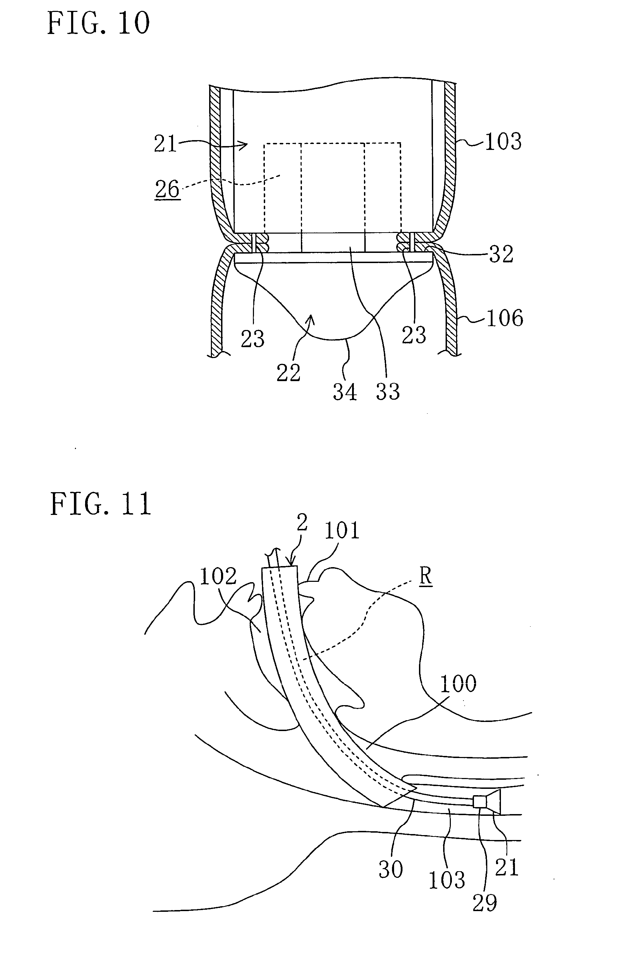

Next, total extirpation of a stomach affected by cancer (not shown) using the above-described supporting device **1** is described. In this operation, the medical instrument is, for example, an anastomosis device **20** as disclosed in Japanese Laid-Open Patent Publication No. 8-66406. Firstly, the structure of the anastomosis device **20** is described with reference to FIG. **7** to FIG. **10.** The anastomosis device **20** includes a main body **21** and a head **22** detachable from the main body **21.** The main body **21** has a generally cylindrical shape, and the outside diameter thereof is about 25 mm. As shown in FIG. **8(a),** the outer periphery of an end face ("primary end face") of the main body **21** is provided with a large number of slits **24** all around, through which metal staples **23** (shown only in FIG. **10)** are thrust out. On the primary end face of the main body **21,** the area inside the circle of slits **24** is provided with an annular cutter **25** (shown only in FIG. **8).** The blade edge of the cutter **25** is approximately coplanar with the primary end face of the main body **21.**

On the primary end face of the main body **21,** the area inside the cutter **25** is provided with a cavity **26** for stowing pieces of organic tissue (described later) cut off by the cutter **25.** The bottom of the cavity **26** extends generally perpendicular to the center line of the main body **21** as shown in FIG. **7.** The central area of the bottom of the cavity **26** is provided with a hole **27** extending in the center line direction of the main body **21.** An engagement bar **28** is inserted and retained in the hole **27.** The engagement bar **28** is made of, for example, a metal material, such as stainless steel, or the like, and is provided so as to extend straight in the center line direction of the main **body 21.** The engagement bar **28** has a pointed tip.

The inside of the main body **21** includes an actuator (not shown) for reciprocating the engagement bar **28** in the center line direction. This actuator has a well-known structure which operates on the supply of electric power. The other end of the main body **21** is provided with a connector **29.** The connector **29** accepts an end of a cord 30 for supplying electric power to the actuator. The anastomosis device **20** has a controller (not shown) for supplying electric power to the actuator. The other end of the cord **30** is connected to the controller. When this controller supplies electric power to the actuator to withdraw the engagement bar **28,** the engagement bar **28** is pulled back and accommodated in the main body **21.** If in this situation the actuator is activated to thrust forward the engagement bar **28,** the engagement bar **28** extends out of the cavity **26.**

The inside of the main body **21** is capable of accommodating a large number of staples **23** correspondingly to the slits **24.** Each staple **23** has a generally U-shape and provided such that the opened part of the shape of "U" faces outward through the slit **24.** Further accommodated in the inside of the main body **21** is a stapler mechanism (not shown) for thrusting out the staples **23** as actuated by the actuator. This stapler mechanism is also a well known mechanism.

The head **22** has a generally umbrella-shape. The head **22** includes a disk member **32** conformable to the shape of the primary end face of the main body **21,** an extended part **33** extending from the center of the disk member **32** in the center line direction, and a mound member **34** elevated on the other side of the disk member **32** with respect to the extended part **33.**

The disk member **32** is made of, for example, a metal material, such as stainless steel, or the like. The disk member **32** has a large number of hollows **35** on its surface facing the main body **21** along the outer perimeter correspondingly to the slits **24** as shown in FIG. **8(b).** These hollows 35 compressively bend an open end of a staple **23** pushed out through the slit **24.** The blade edge of the cutter **25** of the main body **21** abuts on the disk member **32** at an inner position with respect to the circle of hollows 35.

The extended part **33** is formed of the same metal material as that of the disk member **32** in a cylindrical shape. The extended part **33** has an engagement hole **36** on the other side of the disk member **32.** The engagement bar **28** is inserted into the engagement hole **36** for engagement. Specifically, the extended part **33** has an engagement mechanism (not shown) for engaging with longitudinal intermediate part of the inserted engagement bar **28** to prevent the engagement bar **28** from falling out of the engagement hole **36.** The mound member **34** is made of a resin material and is fixed to the disk member **32.**

Next, the procedure of inserting the tubular member **2** of the supporting device 1 into the pharynx **100** is described. This tubular member **2** is inserted after the second guiding member **5** and the first guiding member **4** are inserted into the pharynx **100** of a patient lying face up. Specifically, at the first step, the mouth of the patient is opened and, although not shown, the second guiding member 5 is inserted into the oral cavity **102** with its digestive organ end first and thrust into the pharynx **100** as it is. In this process, tapered part of the second guiding member **5** faces the back side of the patient. The second guiding member **5** is thrust till its digestive organ end reaches the vicinity of the entrance of the esophagus **103.** When the second guiding member **5** is entirely inserted, the pharynx 100 is expanded by the second guiding member **5** so that its inside diameter is increased. The second guiding member **5** is long and has a small diameter as compared with the first guiding member **4** and the tubular member **2** and is therefore insertable into the pharynx **100** relatively readily.

In the process of inserting the second guiding member **5** into the pharynx **100,** the second guiding member 5 can be smoothly inserted while pushing aside tissues blocking the pharynx **100** because the digestive organ end of the second guiding member 5 has a tapered shape.

Thereafter, the first guiding member **4** is inserted into the pharynx **100** with the digestive organ end first. In this case, firstly, the oral cavity end of the second guiding member **5** is inserted into the first guiding member **4,** and the first guiding member **4** is moved toward the digestive organ along the second guiding member **5.** At this step, the first guiding member 4 is guided by the second guiding member 5. The first guiding member **4** is inserted till the digestive organ end of the first guiding member **4** reaches the vicinity of the digestive organ end of the second guiding member **5.**

As the first guiding member **4** is inserted into the pharynx **100,** the cavity **4c** of the first guiding member **4** engages with the protrusion **5b** of the second guiding member **5,** such that the first guiding member **4** becomes immovable with respect to the second guiding member **5.** In this condition, the digestive organ end of the first guiding member **4** is at substantially the same position as the digestive organ end of the second guiding member **5.** Thus, the operator can perceive whether or not the insertion position of the first guiding member **4** is correct with respect to the second guiding member 5 only by engaging the cavity **4c** with the protrusion **5b.**

When the first guiding member **4** is entirely inserted into the pharynx **100,** the second guiding member **5** is covered with the first guiding member **4,** and the pharynx **100** is expanded by the first guiding member 4 so that the inside diameter of the pharynx **100** is further increased.

When the first guiding member **4** is entirely inserted into the pharynx **100,** the oral cavity end of the second guiding member **5** can extend ahead of the oral cavity end of the first guiding member **4** because the second guiding member 5 is longer than the first guiding member **4.** With this arrangement, it is possible to readily hold the second guiding member **5** in fingers and pull out the second guiding member **5** from the oral cavity end of the first guiding member **4.** Further, with the above-described coating on the inner surface of the first guiding member **4** and the outer surface of the second guiding member **5,** it is possible to readily pull out the second guiding member **5.**

Since the diameters of the first guiding member **4** and second guiding member **5** are smaller than the diameter of the tubular member **2,** the work of inserting the first guiding member **4** and the second guiding member 5 into the pharynx **100** is simple as compared with insertion of the tubular member **2** into the pharynx **100.** Since the first guiding member **4** and the second guiding member **5** are made of a resin material only, the first guiding member **4** and the second guiding member 5 do not cause load on the tissue of the oral cavity **102** and pharynx **100.**

After the second guiding member is pulled out of the first guiding member, the tubular member **2** is inserted into the pharynx **100** with the digestive organ end of the tubular member **2** first. In this case, firstly, the oral cavity end of the first guiding member **4** is inserted into the inner passageway **2b** of the tubular member **2,** and then, the tubular member **2** is moved along the first guiding member **4** toward the digestive organ. In this process, the tubular member **2** is guided by the first guiding member **4.** The tubular member **2** is inserted till its digestive organ end reaches the vicinity of the entrance of the esophagus **103.**

In the process of inserting the tubular member **2** into the pharynx **100,** the cavity **2c** of the tubular member **2** engages with the protrusion **4b** of the first guiding member **4,** such that the tubular member **2** becomes immovable with respect to the first guiding member **4.** In this condition, the digestive organ end of the tubular member **2** is at substantially the same position as the digestive organ end of the first guiding member **4.** Thus, the operator can perceive whether or not the insertion position of the tubular member **2** is correct with respect to the first guiding member **4** only by engaging the cavity **2c** with the protrusion **4b.**

When the tubular member **2** is entirely inserted into the pharynx **100** as described above, the first guiding member **4** is covered with the tubular member **2,** and the pharynx **100** is expanded by the tubular member **2** so that the inside diameter of the pharynx **100** is further increased. Thereafter, the first guiding member **4** is pulled out from the oral cavity end of the tubular member **2.** The oral cavity end of the first guiding member **4** can extend ahead of the oral cavity end of the tubular member **2** because the first guiding member **4** is longer than the tubular member **2,** and therefore, it is possible to readily hold the first guiding member 4 in fingers for pulling it out.

When the tubular member **2** is inserted into the pharynx **100,** it is scarcely necessary to greatly deform the tubular member **2** and the reinforcement member **3** because the tubular member **2** has a curved shape conformable to the shape of the pharynx **100.** Therefore, smaller force is sufficient for inserting the tubular member **2** and the reinforcement member **3** into the pharynx **100.** Further, since it is not necessary to deform the tubular member **2** in such a way, the tubular member **2** is free from flattening deformation, so that the shape of the inner passageway **2b** is maintained.

Since the pharynx **100** is expanded by the tubular member **2,** force in flattening direction is imposed on the tubular member **2,** but flattening deformation of the tubular member **2** is suppressed because the tubular member **2** is reinforced by the reinforcement member **3.**

Since the reinforcement member **3** is formed by a thin plate extending along the perimeter of the inner passageway **2b,** the dimension of the reinforcement member **3** in the thickness direction of the peripheral wall **2a,** i.e., the thickness of the thin plate, T, is set smaller than the wire diameter when the conventional reinforcement member is formed by a wire, so that the thickness of the peripheral wall **2a** is decreased, while the width of the thin plate, W, is set greater than the wire diameter of the conventional wire, whereby sufficient strength of the reinforcement member **3** can be obtained. Thus, flattening deformation of the tubular member **2** can be suppressed while the thickness of the peripheral wall **2a** of the tubular member **2** is decreased. Therefore, the size of insertion space R of the medical instrument, which is formed by the inner passageway **2b** of the tubular member **2,** can be maintained to have a sufficient size for the main body **21,** which has a larger diameter than that of a generally-employed endoscope, to pass through.

Since the inside diameter of the pharynx **100** is gradually increased by the second guiding member **5** and the first guiding member 4 whose diameters are smaller than that of the tubular member 2 before the tubular member **2** is inserted into the pharynx **100,** the force necessary for inserting the tubular member **2** can be decreased. Since the tubular member **2** is inserted into the pharynx **100** after the pharynx **100** is gradually expanded in such a way, the load on a patient is decreased as compared with a case where the tubular member **2** is inserted into the pharynx **100** without such a pretreatment.

Meanwhile, the patient is laparotomized. A portion in the vicinity of the border between the esophagus **103** and the stomach and a portion in the vicinity of the border between the small intestine **106** and the stomach are cut, so that the stomach is enucleated.

Then, as shown in FIG. **11,** the main body **21** of the anastomosis device **20** is inserted from the oral cavity end of the tubular member **2** into the inner passageway **2b.** In this process, the cord 30 is pushed into the inner passageway **2b,** whereby the main body **21** is guided by the inner passageway **2b** to the esophagus **103** to reach an area in the vicinity of the open end of the esophagus **103.** Then, the engagement bar **28** of the main body 21 is extended from the open end of the esophagus **103,** and the open end of the esophagus 103 is lashed, and thereby retained, to the basal end of the engagement bar **28** with a thread.

The head **22** is inserted into the small intestine **106** from the open end of the small intestine **106.** The extended part **33** of the head **22** is thrust out of the peripheral wall of the small intestine **106,** and the engagement bar **28** is inserted into and engaged with the engagement hole **36** of the extended part **33.** Thereafter, the controller supplies electric power to the main body **21,** so that the engagement bar **28** is pulled into the main body **21** as shown in FIG. 10, and as a result, the head **22** is in the vicinity of the main body **21** such that the peripheral wall of the esophagus **103** comes in contact with the peripheral wall of the small intestine **106.**

While the head **22** is in the vicinity of the main body **21,** staples **23** are thrust out through the slits **24** of the main body **21.** The staples **23** penetrate through the peripheral wall of the esophagus 103 and the peripheral wall of the small intestine **106** to reach the hollows **35** of the head **22** and are bent by the hollows **35,** whereby the esophagus **103** is anastomosed to the small intestine **106.** At this time of anastomosis, the cutter **25** of the main body **21** abuts on the disk member **32** of the head **22,** and the tissue of the esophagus **103** and small intestine 106 inside the circle of the anastomosed parts of the peripheral wall of the esophagus **103** and the peripheral wall of the small intestine **106** is ablated and stowed in the cavity **26** of the main body **21.** It should be noted that, although not shown, the open end of the small intestine **106** is separately closed using a suture, or the like.

After completion of the anastomosis of the esophagus 103 with the small intestine **106,** the cord **30** extending out from the oral cavity end of the tubular member **2** is pulled, whereby the main body **21** and the head **22** can be pulled out of the esophagus **103** through the inner passageway 2b of the tubular member **2** while the main body **21** and the head **22** are kept integrated with each other. After the main body **21** and the head **22** are pulled out of the esophagus **103,** the tubular member **2** is pulled out of the pharynx **100.**

Thus, according to this embodiment, the tubular member **2** is retained in the pharynx 100, and the main body **21** is guided by the tubular member **2** up to the vicinity of the exit of the esophagus **103.** With such arrangements, the main body **21** can be inserted into the esophagus **103** without incision in the neck or chest. Therefore, in the case where the anastomosis device **20** is used for total extirpation of the stomach, the number of incisions in a patient can be reduced.

Since the reinforcement member **3** is formed by a thin plate extending along the perimeter of the inner passageway **2b** of the tubular member **2,** the thickness of the peripheral wall **2a** of the tubular member **2** can be reduced while flattening deformation of the tubular member **2** is suppressed, so that large insertion space R can be secured for the main body **21.** Since the tubular member **2** and the reinforcement member **3** are curved so as to be conformable to the shape of the pharynx **100,** the force necessary for inserting the tubular member 2 and the reinforcement member **3** into the pharynx **100** is small, and accordingly, the workability of insertion of the tubular member **2** into the pharynx **100** is improved, while the load on a patient can be decreased.

Since the tubular member **2** is inserted into the pharynx **100** after the pharynx **100** is gradually expanded by the first guiding member **4** and the second guiding member **5,** large insertion space R can be secured for the main body **21** while the load on a patient is suppressed, and the workability of insertion of the tubular member **2** into the pharynx 100 is further improved.

Since the resin material of the first guiding member **4** and the second guiding member **5** is harder than the resin material of the tubular member **2,** flattening deformation of the guiding members **4** and **5** dwelling in the pharynx **100** is reduced so that the pharynx **100** can be effectively expanded. Since the tubular member **2,** which has a larger diameter than the first guiding member **4,** is made of a resin material softer than the resin material of the guiding member **4,** the load on a patient can be further reduced.

Since the reinforcement member **3** has a spiral shape, intermediate part of the tubular member **2** in the center line direction can be continuously reinforced, and the shape of the inner passageway **2b** can be maintained in the initial shape between its ends along the center line.

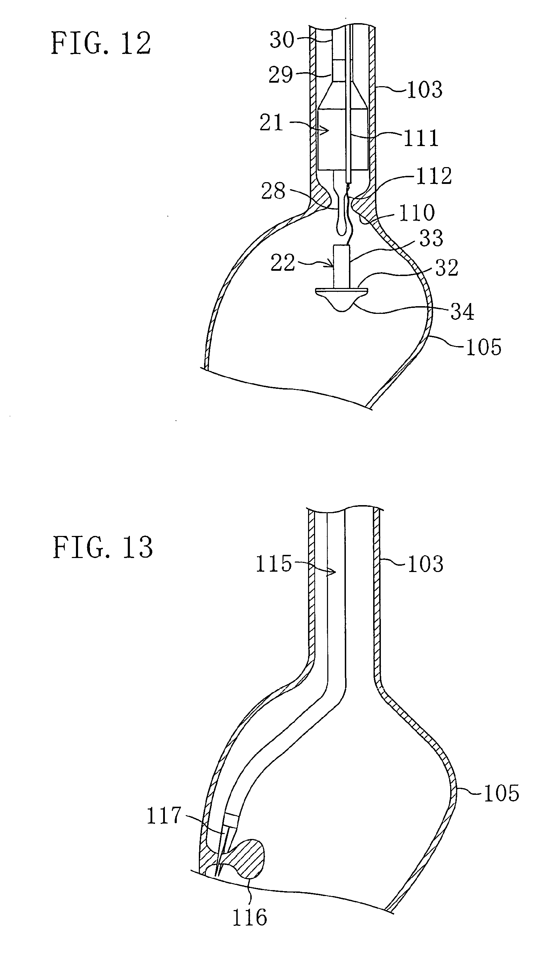

Referring to FIG. **12,** the anastomosis device **20** can also be used for the treatment of a stricture **110** formed in the vicinity of the exit of the esophagus **103.** The stricture **110** consists of a tumor swelling on the inner wall of the esophagus **103** toward the central area of the esophagus **103,** or the like. In the treatment of the stricture **110,** as in total extirpation of the stomach, the tubular member **2** is inserted into the pharynx **100,** and the main body **21** is inserted up to the exit of the esophagus **103** through the tubular member **2.** In this process, a tube **111,** which is thinner than the cord **30** of the main body **21,** is inserted up to the exit of the esophagus **103** through the tubular member **2** as is the main body **21.** The tube **111** includes a thread passing therethrough from the oral cavity end of the tube **111,** which is to be tied to the extended part **33** of the head **22.**

After the laparotomy of the patient, the wall of the stomach 105 is incised, and the head **22** is inserted inside the stomach **105.** Before the insertion of the head **22** into the stomach **105,** an end of a thread **112** is tied to the extended part 33 of the head **22.** After the head **22** is inserted into the stomach **105,** the thread **112** is pulled from the oral cavity side, whereby the extended part **33** of the head **22** is oriented to the main body 21. With this orientation, the engagement bar **28** is inserted into and engaged with the engagement hole **36** of the extended part **33.** Thereafter, the main body **21** is supplied with electric power by a controller, and the engagement bar **28** is pulled back into the main body **21,** so that the head **22** is in the vicinity of the main body **21.** As a result, the stricture **110** of the esophagus **103** is sandwiched by the disk member **32** of the head **22** and the cutter **25** and ablated by the cutter **25.** The ablated part is stowed in the cavity **26** of the main body **21.** Thereafter, the main body **21** and the head **22,** which are still integrated together, are pulled out of the esophagus 103 through the inner passageway **2b** of the tubular member **2.**

In the case where two medical instruments, for example, the main body **21** and the tube **111,** need to be inserted into the esophagus **103** as in the treatment of the stricture **110,** using the supporting device 1 makes the manipulation easier and reduces the load on the patient.

Referring to FIG. **13,** the supporting device 1 of the present invention can be used for removing a tumor **116** produced on the inner wall of the stomach **105** using an ablation instrument **115** insertable into a digestive organ. The ablation instrument **115** is one that has been conventionally used in medical workplaces. The ablation instrument **115** includes a scissors-like blade **117** at the tip, which is the leading end for insertion to a digestive organ, and a manipulator (not shown) at the basal end for manipulating the blade **117.** In the case where the ablation instrument **115** is used to ablate the tumor **116,** the ablation instrument **115** is inserted through the tubular member **2** to pass through the esophagus **103** and reach the stomach **105** in the same way as the main body **21** is in the extirpation of the stomach. The position of the tip end of the ablation instrument **115** can be adjusted by externally manipulating the basal end of the ablation instrument **115** outside the oral cavity **102.** When adjusting the position of the ablation instrument **115,** the ablation instrument **115** itself is moved. However, the ablation instrument **115** moves only in the tubular member **2** but does not scratch the inner wall of the pharynx **100.** Therefore, the load on the patient can be reduced. After the tip of the ablation instrument **115** is positioned in the vicinity of the tumor **116,** the blade **117** is manipulated by the manipulator to ablate the tumor **116.** The ablated tumor **116** is held at the tip of the ablation instrument **115.** The ablation instrument **115** holding the ablated tumor **116** is pulled out of the stomach **105.**

In the operation of ablating the tumor **116** of the stomach **105,** an endoscope (not shown) may be inserted into the stomach **105** in addition to the ablation instrument **115.** In such a case where two medical instruments are inserted in the stomach **105,** using the supporting device **1** makes the manipulation easier and reduces the load on the patient.

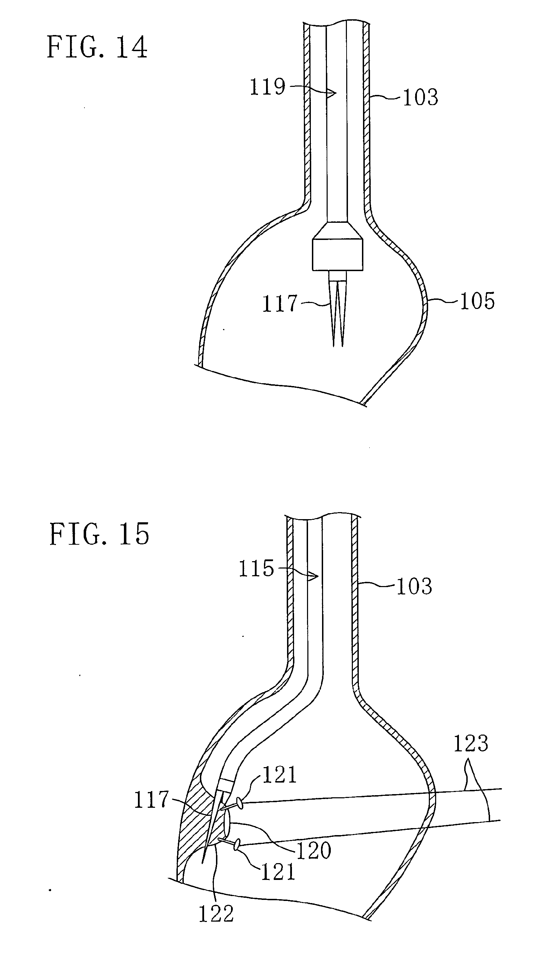

Referring to FIG. **14,** a medical instrument having an endoscope **119** and a small blade **117** integrated therewith may be used to ablate the tumor **116** of the stomach 105 (shown in FIG. **13).** The endoscope **119** is one that has been conventionally used in medical workplaces. An end portion of the endoscope **119,** which is the leading end for insertion, has a larger diameter. In such a case where the endoscope **119** whose end portion has a larger diameter as compared with conventional endoscopes is inserted to the stomach 105, using the supporting device **1** makes the manipulation easier and reduces the load on the patient.

Referring to FIG. **15,** the supporting device **1** of the present invention can also be used in the ablation of early-stage stomach cancer **120** produced on the gastric mucosa of the stomach **105** with the ablation instrument **115** insertable into a digestive organ. In the process of ablating the early-stage stomach cancer 120, a small incision is first made on the abdomen of a patient without laparotomy, and two pins **121** are inserted through this incision in the abdominal cavity using an insertion tool (not shown). These pins **121** pierce through the wall of the stomach **105** to be inserted inside the stomach **105** and stuck in the mucous membrane **122** in the vicinity of the cancer **120.** The pins **121** are provided with threads **123** tied thereto, and the other ends of the threads **123** are out of the abdominal cavity through the incision on the abdomen. The ablation instrument **115** used herein is the same as the ablation instrument **115** used for ablating the tumor **116** of the stomach **105** and is inserted in the stomach **105** in the same way. By pulling the threads **123** to the outside of the abdominal cavity to pull the mucous membrane **122** in the vicinity of the cancer **120** toward the inside of the stomach **105,** the mucous membrane **122** is deformed to the shape of a mound. The mound portion of the mucous membrane **122** is ablated by the blade **117** of the ablation instrument **115,** whereby the early-stage stomach cancer **120** can be ablated.

In general, in the case of the early-stage stomach cancer, the cancer **120** is produced only in the mucous membrane **122** of the stomach **105,** and it is not necessary to ablate the wall of the stomach **105.** Only by, as described above, inserting the ablation instrument **115** into the stomach **105** and forming small openings in the wall of the stomach **105** for passage of the pins **121,** the early-stage stomach cancer **120** can be ablated with less load.

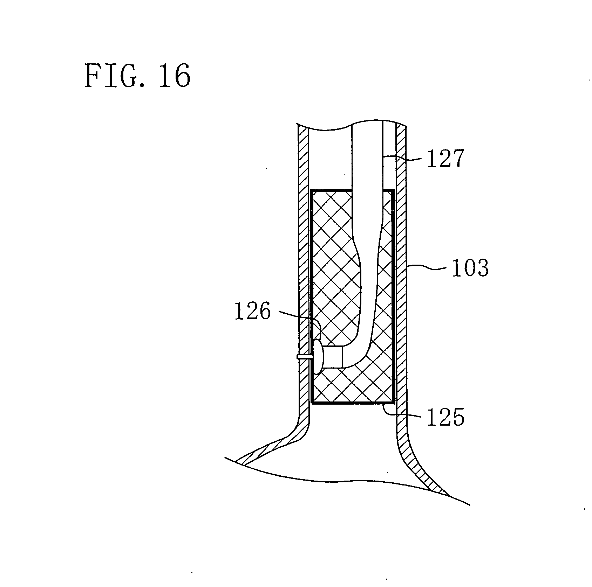

Referring to FIG. **16,** the supporting device **1** of the present invention can be used for installing a stent **125** in the esophagus **103.** The first step of the method for installing the stent **125** is to insert the stent **125** to a position in the esophagus **103** at which the stent **125** is to be installed. Thereafter, a pin **126** for affixing the stent **125** to the inner wall of the esophagus **103** and a stick **127** as the medical instrument for sticking the pin **126** in the esophagus 103 are inserted up to the esophagus **103** through the tubular member **2.** Then, a centesis portion of the pin **126** is oriented from the inside of the stent **125** toward the inner wall of the esophagus **103,** and thereafter, the head of the pin **126** is pushed toward the outside of the esophagus **103** such that the centesis portion of the pin **126** pierces through the stent **125** and is stuck in the esophagus **103.** In this way, the stent **125** can be installed at a predetermined position in the esophagus **103.**

In embodiment 1, the tubular member **2** and the reinforcement member **3** have curved shapes so as to be readily inserted into the pharynx **100.** Therefore, the first guiding member **4** and the second guiding member 5 may be omitted.

### (Embodiment 2)

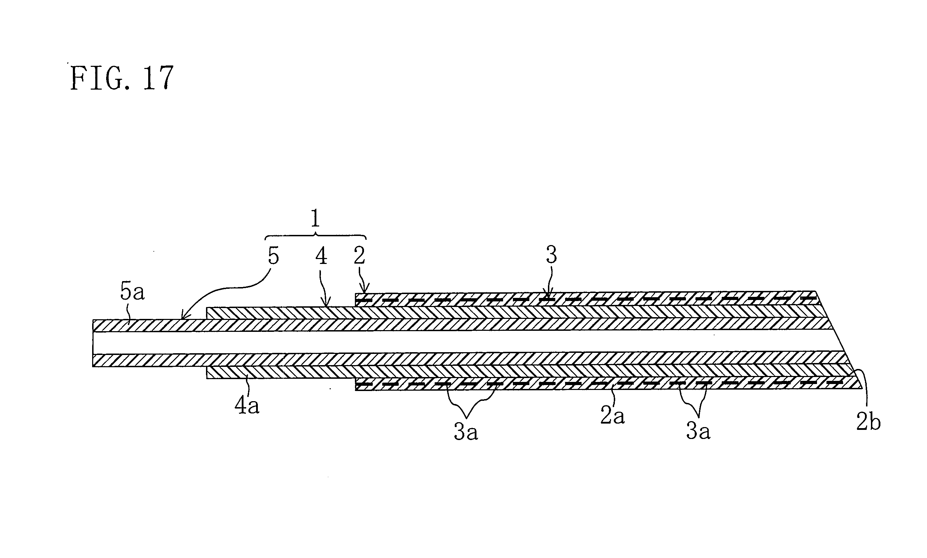

FIG. **17** is a cross-sectional view of a supporting device **1** according to embodiment **2** of the present invention for supporting insertion of a medical instrument into a human body. The supporting device **1** of embodiment **2** is different from, but otherwise the same as, the supporting device **1** in that the shape of the tubular member **2** is linear and that the supporting device 1 of embodiment 2 bears alignment marks. Hereinafter, the same parts as those of embodiment **1** are denoted by the same reference numerals, and the descriptions thereof are omitted, while only the differences are described.

In this embodiment, the tubular member **2** has a linearly elongated shape. Therefore, the reinforcement member **3** insert-molded in the peripheral wall **2a** of the tubular member **2** has such a shape that the center line linearly extends.

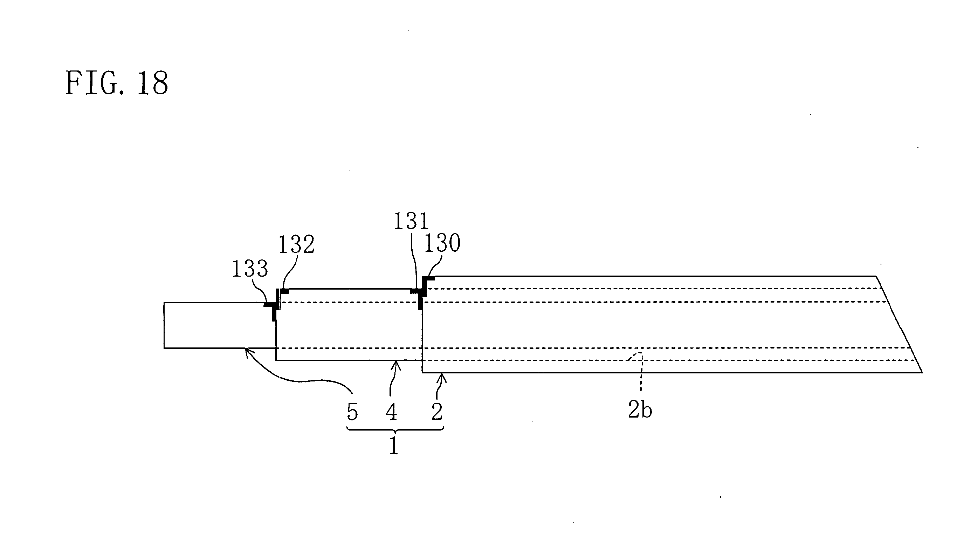

Referring to FIG. **18,** the oral cavity end of the tubular member **2** is provided with a tubular member alignment mark **130.** The tubular member alignment mark **130** is made by applying paint of a color different from that of the resin material of the tubular member **2** on the outer surface of the tubular member **2.** The tubular member alignment mark **130** includes a part extending in the perimeter direction of the tubular member **2** and a part extending in the center line direction of the tubular member **2.**

The oral cavity end of the first guiding member **4** is provided with two first guiding member alignment marks **131** and **132** provided in the center line direction of the guiding member **4** with an interval therebetween. The first guiding member alignment marks **131** and **132** are made by applying paint of a color different from that of the resin material of the first guiding member **4** on the outer surface of the first guiding member **4.** The first guiding member alignment marks **131** and **132** each has a part extending in the perimeter direction of the first guiding member **4** and a part extending in the center line direction of the first guiding member **4.** Among the first guiding member alignment marks **131** and **132,** the alignment mark **131** which is on the digestive organ side is positioned such that, when aligned with the tubular member alignment mark **130,** the digestive organ end of the first guiding member **4** is generally coincident with the digestive organ end of the tubular member **2.** Since the tubular member alignment mark **130** and the first guiding member alignment mark **131** are made on only parts of the perimeter, a predetermined position on the tubular member **2** in the perimeter direction can be made generally coincident with a predetermined position on the first guiding member **4** in the perimeter direction. Thus, tapered part of the digestive organ end of the tubular member 2 can be made generally coincident with tapered part of the digestive organ end of the first guiding member **4.**

The oral cavity end of the second guiding member **5** is provided with a second guiding member alignment mark **133.** The second guiding member alignment mark **133** is made by applying paint of a color different from that of the resin material of the second guiding member **5** on the outer surface of the second guiding member **5.** The second guiding member alignment mark **133** includes a part extending in the perimeter direction of the second guiding member **5** and a part extending in the center line direction of the second guiding member **5.** The second guiding member alignment mark **133** is positioned such that, when aligned with the first guiding member alignment mark **132** which is provided at the oral cavity end of the first guiding member **4,** the digestive organ end of the second guiding member **5** is coincident with the digestive organ end of the first guiding member **4.** The alignment marks **130** to **133** may be formed by a cavity or projection as an alternative to the paint.

The supporting device **1** of embodiment 2 can be used, as can the supporting device 1 of embodiment 1, for total extirpation of a stomach, the treatment of a stricture of the esophagus, ablation of a tumor produced in the inner wall of the stomach, ablation of early-stage stomach cancer, installation of a stent, etc.

Specifically, when the supporting device **1** is used, the inside diameter of the pharynx **100** is gradually increased by the second guiding member **5** and the first guiding member **4** whose diameters are smaller than that of the tubular member **2** before the tubular member **2** is inserted into the pharynx **100.** Therefore, the force necessary for inserting the tubular member **2** having a large diameter, which secures a large insertion space R for the medical instrument, into the pharynx **100** can be decreased. Further, the gradual expansion of the pharynx **100** can decreases the load on a patient.

The first guiding member **4** and the second guiding member **5** have smaller diameters than that of the tubular member **2** and are therefore readily insertable into the pharynx **100.** The first guiding member **4** can guide the tubular member **2** into the pharynx **100** and make insertion of the tubular member **2** easier.

The second guiding member **5** has a smaller diameter and therefore can be readily and correctly inserted to a predetermined position in the pharynx **100.** When inserting the first guiding member **4** into the pharynx **100** along the second guiding member **5,** the digestive organ end of the first guiding member **4** is made coincident with the digestive organ end of the second guiding member **5** by aligning the first guiding member alignment mark **132** with the second guiding member alignment mark **133.** With this, the operator can perceive whether or not the insertion position of the first guiding member **4** is correct with respect to the second guiding member 5 as a reference only by visually checking the alignment marks **132** and **133.**

When inserting the tubular member 2 into the pharynx **100** along the first guiding member **4** in the same way, the digestive organ end of the tubular member **2** can be made generally coincident with the digestive organ end of the first guiding member **4** by aligning the tubular member alignment mark **130** with the first guiding member alignment mark **131.** With this, the operator can perceive whether or not the insertion position of the tubular member **2** is correct with respect to the first guiding member **4** as a reference only by visually checking the alignment marks **130** and **131.** It should be noted that the positions of the alignment marks **130** to **133** can be set arbitrarily. The position of the first guiding member **4** with respect to the second guiding member **5** and the position of the tubular member **2** with respect to the first guiding member **4** can be set according to the positions of the alignment marks **130** to **133.**

Although in embodiments **1** and **2** the reinforcement member **3** is insert-molded in the tubular member **2,** the reinforcement member 3 may be combined with a premolded tubular member **2.**

The supporting device **1** can also be used for inserting a medical instrument other than those described above, such as a catheter, etc., into a digestive organ.

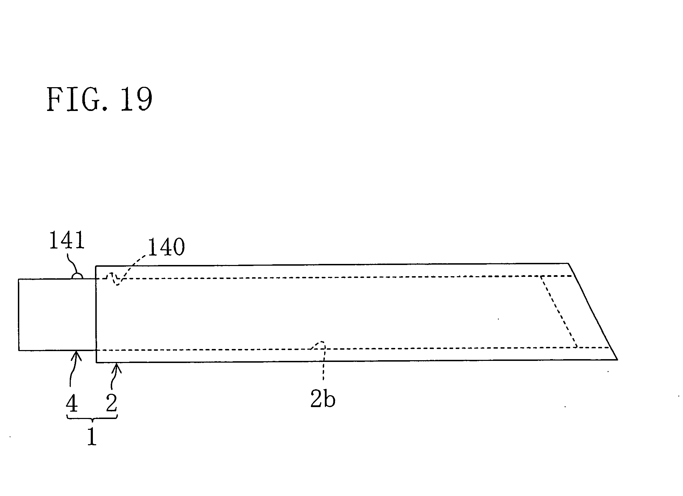

Although in embodiments 1 and 2 the pharynx **100** is gradually expanded by the first guiding member **4** and the second guiding member **5** before the tubular member **2** is inserted into the pharynx **100,** the pharynx **100** may be expanded only by the first guiding member **4** before the tubular member **2** is inserted into the pharynx **100** as in the variation shown in FIG. **19.**

In this variation, the inner surface of the tubular member **2** on the oral cavity side has a cavity 140, and the outer surface of the first guiding member **4** on the oral cavity side has a protrusion **141** fittingly engageable with the cavity **140.** The protrusion **141** is positioned such that, when the protrusion **141** is engaged with the cavity **140,** the digestive organ end of the first guiding member **4** is generally coincident with the digestive organ end of the tubular member **2.** Thus, when inserting the tubular member **2** into the pharynx **100** along the first guiding member **4,** the cavity **140** is engaged with the protrusion **141** so that the tubular member **2** becomes immovable with respect to the first guiding member **4.** In this condition, the digestive organ end of the tubular member **2** can be made generally coincident with the digestive organ end of the first guiding member **4.** With this, the operator can perceive whether or not the insertion position of the tubular member **2** is correct with respect to the first guiding member **4** as a reference only by engaging the cavity **140** with the protrusion **141.** The cavity **140** is a tubular member engagement section of the present invention. The protrusion **141** is a guiding member engagement section of the present invention.

Although not shown, the tubular member engagement section may be formed by a protrusion, and the guiding member engagement section may be formed by a cavity. Alternatively, these engagement sections may have shapes different from the cavity and protrusion. The positions of these engagement sections can be set arbitrarily. The position of the tubular member **2** with respect to the first guiding member **4** can be set according to the positions of these engagement sections.

In the supporting device 1 of embodiment 1, the tubular member **2,** the first guiding member **4** and the second guiding member **5** may be provided with alignment marks and engagement sections as in embodiment 2.

### Industrial Applicability

As described above, a supporting device of the present invention for supporting insertion of a medical instrument into a human body is suitable to insertion of, for example, an anastomosis device for anastomosing digestive organs from the oral cavity into a digestive organ.

## Claims

1. A supporting device for supporting insertion of a medical instrument into a human body, comprising:
a tubular member having an inner passageway between its opposite ends through which the medical instrument is capable of passing; and
a reinforcement member formed by a thin plate extending along a perimeter of the inner passageway,
wherein the tubular member and the reinforcement element have curved shapes conformable to the shape of a pharynx of a human body and, when inserted from an oral cavity into the pharynx and retained there, guide the medical instrument to a digestive organ through the inner passageway.

2. A supporting device for supporting insertion of a medical instrument into a human body, comprising:
a tubular member having an inner passageway between its opposite ends through which the medical instrument is capable of passing;
a reinforcement member extending along a perimeter of the inner passageway; and
a guiding member having a diameter smaller than the inner passageway and insertable from an oral cavity of a human body into a pharynx, the guiding member guiding, when inserted into the pharynx, the tubular member and the reinforcement member from the oral cavity to the pharynx,
wherein, when guided to the pharynx and retained there, the tubular member and the reinforcement member guide the medical instrument to a digestive organ through the inner passageway.

3. The supporting device of claim 1, further comprising a guiding member having a diameter smaller than the inner passageway of the tubular member and insertable from the oral cavity into the pharynx wherein, when inserted into the pharynx, the guiding member guides the tubular member and the reinforcement member from the oral cavity to the pharynx.

4. The supporting device of any one of claims 1 to 3, wherein the reinforcement member has the shape of a spiral continuously extending in a center line direction of the inner passageway.

5. The supporting device of any one of claims 1 to 4, wherein a digestive organ end of the tubular member extends toward a digestive organ ahead of a digestive organ end of the reinforcement member.

6. The supporting device of any one of claims 1 to 5, wherein the digestive organ end of the tubular member is slanted with respect to the center line of the inner passageway.

7. The supporting device of any one of claims 1 to 6, wherein the tubular member is molded with the reinforcement member buried therein.

8. The supporting device of any one of claims 2 to 7, wherein:
the guiding member includes a guiding member engagement section; and
the tubular member includes a tubular member engagement section for engaging with the guiding member engagement section of the guiding member inserted up to a predetermined position in the inner passageway of the tubular member.

9. The supporting device of any one of claims 2 to 7, wherein:
the guiding member has a guiding member alignment mark; and
the tubular member has a tubular member alignment mark aligned with the guiding member alignment mark of the guiding member inserted to a predetermined position in the inner passageway of the tubular member.

10. The supporting device of any one of claims 1 to 9, wherein:
the tubular member is made of a resin material; and
the guiding member is made of another resin material harder than the resin material of the tubular member.
